(19) Europäisches Patentamt
European Patent Office
Office européen des brevets

(11) Publication number : **0 652 290 A1**

(12) # EUROPEAN PATENT APPLICATION

(21) Application number : **94114040.2**

(22) Date of filing : **07.09.94**

(51) Int. Cl.[6] : **C12Q 1/37,** A61K 38/57, A61K 38/48

(30) Priority : **07.09.93 US 117863**
**01.09.94 US 299401**

(43) Date of publication of application :
**10.05.95 Bulletin 95/19**

(84) Designated Contracting States :
**AT BE CH DE DK ES FR GB GR IE IT LI LU MC NL PT SE**

(71) Applicant : **YISSUM RESEARCH DEVELOPMENT COMPANY OF THE HEBREW UNIVERSITY OF JERUSALEM**
**46, Jabotinsky Street,**
**P.O. Box 4279**
**Jerusalem 91042 (IL)**

(72) Inventor : **Ben-Neriah, Yinon**
**5 Mevo, Duvdevan P.O. Box 1691**
**Mevasseret Zion 90805 (IL)**
Inventor : **Alkalay, Irit**
**40 Shevet Biniamin St.**
**Givat Zeev (IL)**

(74) Representative : **VOSSIUS & PARTNER**
**Postfach 86 07 67**
**D-81634 München (DE)**

(54) **Method for regulation of NF-kB.**

(57) Methodology for identifying compositions which affect NF-κB activation via IκBα regulation and uses therefor are described. Also described are IκBα-protease inhibitors useful in the methods of the invention.

EP 0 652 290 A1

This invention relates generally to the field of regulation of gene expression and specifically to the regulation of transcription factor NF-κB through regulation of degradation of the NF-κB inhibitor, IκBα.

The nuclear factor-kappa B (NF-κB) is an inducible transcription factor which participates in the regulation of multiple cellular genes after treatment of cells with factors such as phorbol ester, lipopolysaccharide (LPS), interleukin-1 (IL-1) and tumor necrosis factor α. These genes are involved in the immediate early processes of immune, acute phase, and inflammatory responses. NF-κB has also been implicated in the transcriptional activation of several viruses, most notably the type 1 human immunodeficiency virus (HIV-1) (Nabel, *et al., Nature*, 326:711, 1987; Kaufman, *et al., Mol. Cell. Biol.*, 7:3759, 1987).

NF-κB is a dimeric transcription factor that binds and regulates gene expression through decameric cis-acting κB DNA motifs. Although a p50/p65 heterodimer has traditionally been referred to as NF-κB and remains the prototypical and most abundant form, it has been recognized recently that several distinct but closely related homo- and heterodimeric factors are responsible for κB site-dependent DNA binding activity and regulation. The various dimeric factors are composed of members of the family of Rel-related polypeptides. One subclass of this family, distinguished by its proteolytic processing from precursor forms and lack of recognized activation domains, includes p50 (NFKB1) and p50B (NFKB2, p52), whereas the second subclass contains recognized activation domains and includes p65 (RelA), RelB, c-Rel, v-Rel, and the *Drosophila* protein Dorsal. All Rel-related members share a 300-amino acid region of homology, responsible for DNA binding and dimerization, called the Rel homology domain. In the cytoplasm, NF-κB and Rel proteins form a "Rel complex".

Activation of the NF-κB transcription factor and various related forms can be initiated by a variety of agents, including TNFα, phorbol 12-myristate 13-acetate (PMA), interleukin-1 (IL-1) and interleukin-2 (IL-2). Activation proceeds through a post-translational event in which preformed cytoplasmic NF-κB in the Rel complex is released from a cytoplasmic inhibitory protein, IκBα. IκBα inhibits transactivation of the p50/p65 heterodimer, by binding to the p65 component, blocking the dimer's translocation to the nucleus. IκBα also inhibits complexes containing c-Rel or RelB. IκBα blocks binding of various NF-κB dimers to κB binding sites in DNA *in vitro*.

Phorbol ester and TNFα induction of nuclear localization of NF-κB is associated with both the degradation of preformed IκBα and the activation of IκBα gene expression. Transfection studies indicate that the IκBα gene is specifically induced by the 65-kilodalton transactivating subunit of NF-κB (Sun, *et al., Science,* 259:1912, 1993). Association of the newly synthesized IκBα with p65 restores intracellular inhibition of NF-κB DNA binding activity and prolongs the survival of this labile inhibitor. These studies show that NF-κB controls the expression of IκBα by means of an inducible autoregulatory pathway.

NF-κB gene regulation is involved in many pathological events including progression of acquired immune deficiency disease (AIDS), the acute phase response and the activation of immune and endothelial cells during toxic shock, allograft rejection, and radiation responses. Recently, cell-free experiments alluded to the possibility that IκBα phosphorylation might play a role in the inactivation and release of the inhibitor (Ghosh, *et al., Nature*, 344:678, 1990) and transient IκBα phosphorylation has been observed in several *in vivo* activation studies (Brown, *et al., Proc. Natl. Acad. Sci., U.S.A.*, 90:2532, 1993; Beg, *et al., J. Mol. Cell. Biol.,* 13:3301,1993). Surprisingly, for the first time the present invention unequivocally establishes that it is not phosphorylation of IκBα that causes dissociation of IκBα from the Rel complex, but rather the phosphorylation renders it susceptible to proteolysis.

Therefore, identification of the nature of IκBα protease is critical for identification of specific inhibitors of the protease which would be effective as anti-inflammatory and immunosuppressive agents. The present invention provides methodologies for identifying such inhibitors and provides a group of inhibitors as well.

The present invention is based on the seminal discovery that the enzyme responsible for degradation of IκBα is a chymotrypsin-like serine protease which recognizes phosphorylated IκBα. Because of this finding, the invention provides a method of identifying a composition which affects degradation of IκBα comprising incubating components including the composition, IκBα, and a chymotrypsin-like serine protease under conditions sufficient to allow the components to interact and measuring the effect on the protease caused by the composition. The invention also includes a method of identifying a composition which affects degradation of IκBα comprising incubating components comprising the composition, an inducer of NF-κB, and an indicator cell, and detecting NF-κB activity.

The invention also includes a method of treating an immunopathological disorder associated with NF-κB gene activation in a subject, comprising administering to the subject a therapeutically effective amount of an inhibitor of a chymotrypsin-like serine protease which degrades IκBα.

FIGURE 1a shows a Western blot analysis of the effect of phorbol 12-myristate 13-acetate (PMA)-treatment on IκBα in 70Z/3 pre-B cells.

FIGURE 1b shows an autoradiogram of DNA from PMA treated 70Z/3 pre-B cells probed with $^{32}P$-labeled DNA encompassing the NF-κB binding motif of the mouse κ light chain enhancer.

FIGURE 1c shows the effect of IL-1, LPS and TNF treatments on the activation of NF-κB and stability of IκBα in 70Z/3 cells and HeLa cells. Sections of Western blots and EMSAs (fluorograms from native gels) are shown.

FIGURE 2a shows EMSA (upper panel) and a Western blot (lower panel) of the effect of cyclohexamide on the stability of IκBα and the activation of NF-κB in 70Z/3 cells after PMA stimulation.

FIGURE 2b shows a densitometric quantitation of IκBα after Western blot analysis of cells treated with cyclohexamide alone (left panel) or treated with PMA for the indicated times after 1 hour pre-treatment with cyclohexamide (right panel).

FIGURE 3a shows the effect of p-tosyl-L-phenylalanine chloromethylketone (TPCK) on the induction by PMA on the activation of NF-κB and stability of IκBα in 70Z/3 cells.

FIGURES 3b and 3c show the effect of TPCK on the activation of NF-κB by IL-1, LPS, and PMA in 70Z/3 cells.

FIGURE 3d shows the effect of pyrrolidinedithiocarbamate (PDTC) on the activation of NF-κB and stability of IκBα in 70Z/3 cells.

FIGURE 4a shows NF-κB activity as determined by HIV-LTR-Luc activity. Jurkat cells were stimulated with the following reagents: (P), PMA (phorbol-12 myrislated 13-acetate), 10 ng/ml; (I), A23187 $Ca^{++}$ ionophore, 1μM; (3), anti-CD3 (OKT3), 5μg/ml; (28) anti-CD28 (monoclonal antibody 9.3), 3 μg/ml; (P/I), PMA + $Ca^{++}$ ionophore in IκBα transfected Jurkat cells; (NS) non-stimulated (0.1%, DMSO only).

FIGURE 4b shows an electromobility shift assay (EMSA) of the effects of various stimuli on NF-κB activity.

FIGURE 4c is a Western blot showing phosphorylation of IκBα after stimulation of cells with various stimuli.

FIGURE 5a shows kinetics of modification of IκBα after PMA and $Ca^{++}$ ionophore (P/I) stimulation.

FIGURE 5b shows IκBα degradation after treatment of cells with NF-κB blockers, TPCK, PDTC and CsA.

FIGURE 6a shows an EMSA of cells treated with N-acetyl-Leu-Leu-Norleucinal (AcLLnL) and the effect on IκBα phosphorylation and degradation.

FIGURE 6b shows an EMSA of the AcLLnL-treated later time points and the effect on NF-κB activity.

FIGURE 6c shows Western blot analysis of immunoprecipitation of Rel A and IκBα after AcLLnL treatment of cells.

FIGURE 7 shows the effects of various peptide aldehyde inhibitors on IκBα. degradation following TNFα, IL-1 or PMA stimulation of cells.

FIGURE 8 shows the effect of various peptide aldehyde inhibitors on NF-κB promoter.

FIGURE 9 shows an EMSA and Western blot of dose response of NF-κB DNA binding and IκBα degradation of benzyloxycarbonyl-Leu-Leu-phenylalaninal (Z-LLF) and AcLLnL.

The present invention provides a method of identifying a composition which affects degradation of IκBα. This method can be accomplished by incubating components comprising the composition, IκBα, and a chymotrypsin-like serine protease under conditions sufficient to allow the components to interact and measuring the effect on the protease caused by the composition.

The NF-κB transcription factor complex is sequestered in the cytoplasm by the inhibitory protein, IκBα. Various cellular stimuli relieve this inhibition by mechanisms which are mostly unknown, leading to NF-κB nuclear localization and transactivation of its target genes. Thus, NF-κB and IκBα are involved in a tightly controlled mechanism of regulation.

The present invention describes the protease which degrades IκBα as a chymotrypsin-like serine protease and describes inhibitors of the protease. According to the invention, the surprising discovery that the IκBα protease is a chymotrypsin-like serine protease provides a useful screening tool for compositions which affect the protease.

Thus, the invention provides a method for identifying compositions which affect degradation of IκBα, such as by inhibiting or stimulating the protease, and thus may be effective as anti-inflammatory and immunosuppressive drugs. The method comprises incubating components, which include the composition to be tested, phosphorylated IκBα and the protease, under conditions sufficient to allow the components to interact, then subsequently measuring the effect the composition on the protease. The observed effect on the protease may be either inhibitory or stimulatory. For example, a composition which inhibits the protease will prevent the protease from degrading IκBα, thereby prohibiting NF-κB from being translocated to nucleus and inhibiting transactivation of genes by NF-κB. The effect the composition has on the stability of IκBα can be determined by various methodologies including immunological, nucleic acid and protein analyses. The IκBα can be labeled so that its fate can be determined. Examples of labels include a radioisotope, a fluorescent compound, a bioluminescent compound, a chemiluminescent compound, a metal chelator or an enzyme. Those of ordinary skill in the art will be able to ascertain such, using routine experimentation.

The method of the invention for identifying a composition which affects degradation of IκBα may further include the addition of NF-κB, preferably bound to or complexed with IκBα. The chymotrypsin-like serine protease which degrades IκBα may be chymotrypsin. A candidate cytoplasmic protease which can degrade IκBα following stimulation is a ubiquitous 700kD multisubunit proteosome which has chymotrypsin-like activity (Vinitsky, *et al., Biochemistry*, 31:9421, 1992). A proteosome as described herein is a multicatalytic enzyme complex.

The invention also includes a method of identifying a composition which affects degradation of IκBα comprising incubating components including the composition to be tested, an inducer of NF-κB and an indicator cell, and detecting NF-κB activity. The inducer of NF-κB can be added prior to or following the addition of the composition to be tested. Preferably, it is added after the composition is added. Inducers of NF-κB include IL-1, IL-2, TNFα, phorbol ester and LPS. Other inducers will be known to those of skill in art.

The method of the invention is performed in an indicator cell. An "indicator cell" is one in which activation of NF-κB can be detected. Examples of mammalian host indicator cells include the pre-B cell line, 70Z/3, Jurkat T, COS, BHK, 293, CHO, HepG2, and HeLa cells. Other cell lines can be utilized as indicator cells, as long as the level of NF-κB can be detected. The cells can be recombinantly modified to contain an expression vector which encodes one or more additional copies of the κB binding motif, preferably operatively linked to a reporter gene. The cells can also be modified to express IκBα and NF-κB. Preferably, the expression vector which encodes NF-κB, contains the coding region for the p65 subunit of NF-κB, to which IκBα binds.

The host cell may be a yeast modified by recombinant DNA to express NF-κB, a chymotrypsin-like gene derived from a cDNA expression library and κB motif linked to a reporter gene. The expression of the chymotrypsin-like protease would result in IκB degradation and activation of NF-κB, hence induction of reporter activity. In the presence of the composition, the chymotrypsin-like protease would be inhibited resulting in no reporter activity. Examples of markers typically used in yeast reporter studies include β galactosidase (β-gal), HIS3 and LEU2 nutrient selection markers.

The reporter gene is a phenotypically identifiable marker for detection of stimulation or inhibition of NF-κB activation. Markers preferably used in the present invention include the LUC gene whose expression is detectable by a luciferase assay. Examples of markers typically used in prokaryotic expression vectors include antibiotic resistance genes for ampicillin (β-lactamases), tetracycline and chloramphenicol (chloramphenicol acetyltransferase). Examples of such markers typically used in mammalian expression vectors, which are preferable for the present invention, include the gene for adenosine deaminase (ADA), aminoglycoside phosphotransferase (neo, G418), dihydrofolate reductase (DHFR), hygromycin-B-phosphotransferase (HPH), thymidine kinase (TK), xanthine guanine phosphoribosyltransferse (XGPRT, gpt) and β-galactosidase (β-gal).

Transformation of a host cell with recombinant DNA may be carried out by conventional techniques which are well known to those skilled in the art.

Where the host is prokaryotic, such as *E. coli*, competent cells which are capable of DNA uptake can be prepared from cells harvested after exponential growth phase and subsequently treated by the $CaCl_2$ method by procedures well known in the art. Alternatively, $MgCl_2$ or RbCl can be used. Transformation can also be performed after forming a protoplast of the host cell or by electroporation.

When the host is a eukaryote, which is preferable in the method of the invention, such methods of transfection of DNA as calcium phosphate co-precipitates, conventional mechanical procedures such as microinjection, electroporation, insertion of a plasmid encased in liposomes, or virus vectors may be used. Eukaryotic cells can also be cotransformed with DNA sequences encoding the polypeptides of the invention, and a second foreign DNA molecule encoding a selectable phenotype, such as the herpes simplex thymidine kinase gene. Another method is to use a eukaryotic viral vector, such as simian virus 40 (SV40) or bovine papilloma virus, to transiently infect or transform eukaryotic cells and express the protein. (*Eukaryotic Viral Vectors*, Cold Spring Harbor Laboratory, Gluzman ed., 1982).

In the present invention, κB binding motif polynucleotide sequences, preferably operatively linked to a reporter gene, IκBα and NF-κB polynucleotide sequences may be inserted into recombinant expression vectors. The term "recombinant expression vector" refers to a plasmid, virus or other vehicle known in the art that has been manipulated by insertion or incorporation of the genetic sequences. Such expression vectors contain a promoter sequence which facilitates the efficient transcription of the inserted genetic sequence of the host. The expression vector typically contains an origin of replication and a promoter, as well as specific genes which allow phenotypic selection of the transformed cells. Vectors suitable for use in the present invention include, but are not limited to the T7-based expression vector for expression in bacteria (Rosenberg *et al., Gene* 56:125, 1987), the pMSXND expression vector for expression in mammalian cells (Lee and Nathans, *J. Biol. Chem.* 263:3521, 1988) and baculovirus-derived vectors for expression in insect cells. The DNA segment can be present in the vector operably linked to regulatory elements, for example, a promoter (e.g., T7, metallothionein I, or polyhedron promoters).

Detection of NF-κB activity in the method of the invention can be detected by measuring the level of the gene product of the reporter gene. Methods of detection may be immunological, by nucleic acid analysis, by

protein analysis, by nutrient selection, or by enzymatic assay, for example. Other common methods will be known to those of skill in the art.

In another embodiment, the invention provides a method of treating an immunopathological disorder associated with NF-κB gene activation in a subject. Preferably, the immunopathological disorder is associated with TNF, IL-1 or IL-6 production. The method comprises administering to the subject a therapeutically effective amount of an inhibitor of a chymotrypsin-like serine protease which degrades IκBα. The term "immunopathological disorder" refers to any disease which involves the immune response or immunity in general. "Therapeutically effective" as used herein, refers to that amount of inhibitor that is of sufficient quantity to ameliorate the cause of the NF-κB disorder. "Ameliorate" refers to a lessening of the detrimental effect of the disorder in the patient receiving the therapy. The subject of the invention is preferably a human, however, it can be envisioned that any animal with a NF-κB disorder can be treated by the method of the invention, for example, a SCID mouse grafted with human bone marrow (humanized SCID). Examples of immunopathological disorders which can be treated by the method of the invention include acquired immunodeficiency disorder (AIDS), toxic shock syndrome, allograft rejection, ultraviolet and radiation responses, and disorders associated with the activation of T cells, B cells and macrophages during the immune response and the acute phase response and disorders associated with advanced cancer such as tumor necrosis factor-mediated cachexia.

The invention includes a method of modulating the activation of a virus associated with NF-κB transactivation comprising administering to the subject a modulating effective amount of an inhibitor of a chymotrypsin-like serine protease which degrades IκBα. The term modulates refers to either inhibiting or stimulating the activation of a virus. Any virus which is transactivated by NF-κB is included, for example, human immunodeficiency virus (HIV). Essentially, any disorder which is etiologically linked to NF-κB/IκBα would be considered susceptible to treatment.

The present invention provides inhibitors of the protease which are peptide aldehydes. The peptide aldehyde preferably has the formula:

$$R_1R_2LLR_3\text{-CHO}$$

wherein $R_1$ is an optional amine protective group; L is leucine; $R_2$ is optional and has the formula

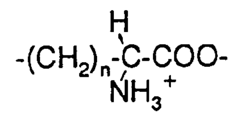

wherein

n is 1-5 and wherein $(CH_2)_n$ is a branched or straight chain alkyl; and $R_3$ is an optional hydrophobic amino acid, except for methionine. The amine protective group is preferably N-acetyl, benzyl-carbonyl or butyl. Examples of such peptide aldehyde inhibitors include proteosome inhibitors such as N-acetyl-Leu-Leu-Norleucinal (AcLLnL) and benzyloxycarbonyl-Leu-Leu-Phenylalaninal (ZLLF). Other peptide aldehydes are useful as inhibitors in the methods of the invention include N-acetyl-Ala-Leu-Leu-Norleucinal (AcALLnL). Most preferred are those peptide aldehydes where the amino acid position $R_3$ is substituted with a hydrophobic amino acid. Examples of $R_3$ include alanine valine, leucine, isoleucine, proline and phenylalanine. Least preferred is a peptide aldehyde wherein the $R_3$ is a charged amino acid. Most preferably, the inhibitor is benzyloxycarbonyl-Leu-Leu-Phenylalaninal (ZLLF).

The inhibitor of the invention can be administered parenterally by injection or by gradual infusion over time. The inhibitor can be administered intravenously, intraperitoneally, intramuscularly, subcutaneously, intracavity, transdermally, or extracorporeally. Methods for delivery of the inhibitor also include orally, by encapsulation in microspheres or proteinoids, by aerosol delivery to the lungs, or transdermally by iontophoresis or transdermal electroporation. Other methods of administration will be known to those skilled in the art.

Preparations for parenteral administration of an inhibitor of the invention include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

In another embodiment, the invention provides a pharmaceutical composition for inhibiting a protease which degrades IκBα, comprising a therapeutically effective amount of an inhibitor of a chymotrypsin-like serine protease which degrades IκBα. The inhibitor is preferably a peptide aldehyde and has the formula:

$R_1R_2LLR_3$-CHO

wherein $R_1$ is an optional amine protective group; L is leucine; $R_2$ is optional and has the formula

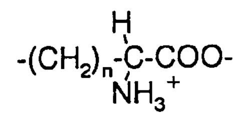

wherein

n is 1-5 and wherein $(CH_2)_n$ is a branched or straight chain alkyl; and $R_3$ is an optional hydrophobic amino acid, except for methionine.

Preferably $R_3$ is selected from the group consisting of alanine, valine, leucine, isoleucine, proline and phenylalanine. The amine protective group is preferably selected from N-acetyl, benzyloxycarbonyl and butyl. Examples of peptide aldehyde inhibitors useful in the pharmaceutical composition of the invention includeN-acetyl-Leu-Leu-Norleucinal(AcLLnL),N-acetyl-Ala-Leu-Leu-Norleucinal (AcALLnL), and benzyloxycarbonyl-Leu-Leu-phenylalaninal (ZLLF). A therapeutically effective amount of inhibitor is that amount that inhibits IκBα degradation by greater than 50% and preferably greater than 80%.

The following examples are intended to illustrate but not limit the invention. While they are typical of those that might be used, other procedures known to those skilled in the art may alternatively be used.

## EXAMPLES

The following examples show that T-cell activation results in the phosphorylation of IκBα and that this is a rapid physiological event that is dependent on appropriate lymphocyte costimulation. The fact that the inducible phosphorylation is abolished by several distinct NF-κB blocking reagents, indicates its essential role in the activation process. A key discovery was that *in vivo* IκBα phosphorylation does not cause its dissociation from the Rel complex, but rather renders IκBα susceptible to proteolysis by the chymotrypsin-like enzyme.

## EXAMPLE 1

### FATE OF IκBα AFTER NF-κB ACTIVATION

The fate of IκBα after treatment of cells with various NF-κB-activating stimuli was investigated. 70Z/3 cells were cultured in RPMI-1640 medium (Gibco BRL) supplemented with 10% fetal calf serum and 50μM 2-mercaptoethanol. Two-ml aliquots with approximately 2.3 x $10^6$ suspended cells were treated with 50 ng/ml$^{-2}$ PMA (Sigma), 50 U/ml IL-1β (Boehringer-Mannheim) or 15 μg/ml LPS (Sigma) for various times. HeLa cells were cultured in DMEM supplemented with 10% fetal calf serum and 1% L-glutamine, and treated with 200 U/ml recombinant human TNF-α (Genzyme). Stimulation was stopped by cooling on ice and immediate centrifugation of cells for 5 sec in an Eppendorf microfuge. Cell pellets ($10^6$ cells) were lysed with 60 μl of a high-salt extraction buffer (Baeuerle, P.A., *et al., Science*, 242:540-546, 1988). Supernatants of lysates (from a 15-min. centrifugation at 13,000 rpm in an Eppendorf Microfuge) were used for analysis by both Western blotting and electrophoretic mobility shift assay (EMSA), as described (Zabel, *et al., EMBO J.*, 12:201, 1983). For SDS-PAGE and Western blotting, 30 μl aliquots of extracts were mixed with 15 μl of SDS sample buffer (Schreck, R., *et al., J. Exp. Med.* 175:1181, 1992), boiled and subjected to SDS-PAGE on 12.5% poyacrylamide mini gels (Biorad).

Proteins were transferred from gels onto Immobilon TM filters (0.45 μm; Millipore) using a semi-dry blotting device (Biorad) for 1 hour at 15V/2.5 mA/cm$^2$. Blotting efficiency was monitored by protein staining of filters with Ponceau S (Serva). Filters were blocked over-night in Tris-buffered saline containing 0.1% (v/v) Tween-20 (TBST), 5% skim milk powder (Nestle) and 1% BSA. The filter was then incubated for 1 hour at room temperature with anti-IκBα IgG in a dialyzed eluate from the IκBα affinity column diluted 1:100 in blocking buffer. After 30 minutes of washing in TBST, the filter was incubated in a 1:4000 dilution of goat anti-rabbit IgG/horse radish peroxidase conjugate (Biorad) in blocking buffer. After 30 minutes of washing in TBST, the filter was treated with ECL detection reagent (Amersham) and exposed to Kodak XR film for less than 1 min. For EMSA, 2 μl aliquots of extracts were added to a binding mix containing 100 mM KCl, 20 mM HEPES, pH 7.9, 2.5 mM dithiothreitol, 0.5 mM phenylmethyl sulfonylfluoride (PMSF), 0.2% Nonidet P-40, 5% Ficoll, 20 μg BSA, 3 μg poly(dI-dC) and 10,000 c.p.m. (Cerenkov counting) of a $^{32}$P-labeled double-stranded κB oligonucleotide probe (Gibco BRL). Specificity of the protein-DNA complex was verified by immunoreactivity with a polyclonal antibody specific for Rel A. After a 20 min-incubation on ice, samples were subjected to electrophoresis on native 4% polyacrylamide gels run in 0.5x TBE. Dried gels were exposed to Kodak XR film at -70°C overnight. Human IκBα was produced in *E. coli* and purified as described. Two milligrams of 6 x His-tagged, purified human IκBα produced in *Escherichia coli* (Zabel, *et al., EMBO J., supra*; Henkel, *et al., Cell*, 68:1121, 1992) were coupled to 0.5 ml cyanogen bromide-activated sepharose 4CL-B (Pharmacia), according to the instruction of the manufacturer. Specific IgG in 2 ml rabbit antiserum raised against IκBα (Zabel, *et al., supra*) was affinity-purified (Henkel, *et al., supra*) on IκBα-sepharose. After extensive washes with phosphate-buffered saline and 2 column volumes of 0.1M glycine-HCl, pH 2.7, specific antibodies were eluted with 2 volumes of 4M guanidinium hydrochloride. The eluate was extensively dialyzed against Tris-buffered saline and used for Western blotting at a dilution of 1:100 in blocking buffer (Henkel, *et al., supra*).

Cells were treated with PMA for indicated periods of time (FIGURE 1a, lanes 2-9). Proteins in total cell extracts were separated by SDS-PAGE and transferred onto a membrane filter followed by Western blot analysis using anti-IκBα IgG and decoration with peroxidase-labelled anti-rabbit IgG. The arrow indicates the position of IκBα. FIGURE 1b shows the effect of PMA on the DNA-binding activity of NF-κB. Total cell extracts from control (lane 1) and PMA-treated cells (lanes 2-6) were incubated with a $^{32}P$-labelled DNA probe encompassing the NF-κB-binding motif from the mouse κ light chain enhancer (Sen, *et al., Cell* 47:921-928, 1986) followed by analysis of DNA-binding activities using electrophoretic mobility shift assay (EMSA). A fluorogram of a native gel is shown. FIGURE 1c shows the effect of IL-1β, LPS and TNF on the activation of NF-κB and stability of IκBα. 70Z/3 cells (upper and middle panels) or HeLa cells (lower panel) were treated with IL-1β (lanes 2-4), LPS (lanes 6-8) or TNF-α (lanes 10-13) for the indicated periods of time followed by analysis of total cell extracts for NF-κB-DNA complex is shown by a filled arrowhead, the position of uncomplexed DNA probe by an open arrowhead and the position of the IκBα band by an arrow.

In extracts from unstimulated 70Z/3 pre-B cells, IκBα-specific IgG detected a single 38K band on Western blots (FIGURE 1a, lane 1) which was not seen with the second antibody alone. Between 2 and 5 minutes after the addition of phorbol 12-myristate 13-acetate(PMA) to cell cultures, IκBα almost completely disappeared from cells (FIGURE 1a, compare lanes 3 and 4). The specific immunoreactivity reappeared after 40 minutes (FIGURE 1a, lane 7). The disappearance of IκBα coincided with the appearance of NF-κB DNA-binding activity in total cells extracts (FIGURE 1b, compare lanes 2 and 3), suggesting a causal relationship between the two events. Interleukin (IL-1β), lipopolysaccharide (LPS) and tumor necrosis factor-α (TNF-α) all induced a decay of IκB in 70Z/3 or HeLa cells (FIGURE 1c), showing that distinct stimuli induced the same reaction. Although most of the IκBα had already decayed 5 minutes after stimulation with PMA, TNF-α and IL 1β, it took more than 15 minutes before there was a rapid decay of the inhibitor in LPS-stimulated 70Z/3 cells. Despite these kinetic differences, the depletion of IκBα was in each case coincident with the appearance of NF-κB activity. In contrast to another study (Brown, *et al., Proc. Natl. Acad. Sci., U.S.A.*, 90:2532, 1993), no transient change in the electrophoretic mobility of IκBα was apparent after induction with the various stimuli, even when phosphatase inhibitors were included in the lysis buffer. Moreover, no breakdown products of IκBα could be detected. Because a polyclonal antibody was used, it is unlikely that only one epitope of IκBα was lost or modified upon stimulation.

## EXAMPLE 2

### EFFECT OF A PROTEIN SYNTHESIS INHIBITOR ON THE STABILITY OF IκBα

70Z/3 cells were treated with 25 μg/ml cycloheximide (Sigma). The protein synthesis inhibitor cycloheximide (CHX) activates NF-κB in 70Z/3 cells. At 10 μg/ml, protein synthesis is inhibited to 90% in 70Z/3 cells. Cell culture, extract preparation, EMSA, SDS-PAGE and Western blotting were as described above in Example 1. Densitometric scanning was done with a Howtek Scanmaster 3 and data analyzed by the software Quantity One Version 2.2.

In FIGURE 2a, 70Z/3 cells were treated with cycloheximide (CHX) for 1 hour followed by stimulation with PMA for the indicated periods of time. Total cell extracts were assayed for NF-κB activity by EMSA (upper panel). A section of an autoradiogram from a native gel is shown with a filled arrowhead indicating the position of the NF-κB-DNA complex. Aliquots of the cell extracts were assayed for IκBα by Western blotting (lower panel). The position of the IκBα band is indicated by an arrow. FIGURE 2b shows the half-life of IκBα in protein synthesis-arrested cells. Cells were treated with CHX alone (left panel) or, after a 1 hour treatment with CHX, induced with PMA for the indicated times (right panel). Total cell extracts of equal protein content were analyzed by western blotting followed by densitometric quantitation of the 38K IκBα band in fluorograms. The amounts of IκBα detected with CHX treatment alone (left panel; open triangles) and after PMA stimulation (right panel, filled triangles) are shown on a half-logarithmic plot. Dotted lines indicate the portion of the slopes used for

estimation of t 1/2 values. The dashed line in the right panel indicates the decay of IκBα in the absence of PMA, as determined in the left panel.

Following a 1 hour treatment of 70Z/3 cells with CHX, the DNA binding of NF-κB was induced only weakly (FIGURE 2a, lane 1, upper panel), and significant levels of IκBα were still present (lower panel). This shows that interference with the normal turnover rate of IκBα is not sufficient for a rapid activation of NF-κB. When CHX-treated cells were stimulated with PMA, a rapid decay of IκBα and further induction of NF-κB binding activity were observed with kinetics indistinguishable from those observed in the absence of CHX (compare FIGURE 2a with FIGURE 1a). But CHX prevented the reappearance of IκBα seen after a 40 minutes treatment with PMA alone (compare FIGURE 2a with FIGURE 1a), consistent with the finding that IκBα is newly synthesized under transcriptional control by NF-κB. A half-life of approximately 138 minutes was determined for IκBα in CHX-treated 70Z/3 cells (FIGURE 2b). If protein synthesis-arrested cells were stimulated with PMA, the half-life of IκBα was reduced to only 1.5 minutes during the phase of its fastest decay, showing that PMA induced a two orders of magnitude decrease in the half-life of IκBα.

The functional significance of the inducible decay of IκBα for activation of NF-κB was tested by exposure of cell cultures to various protease inhibitors. Six distinct serine protease inhibitors with chymotrypsin-like specificity efficiently prevent the induction of NF-κB DNA-binding activity in response to PMA and TNF-α (Table 1). Constitutive DNA-binding activities, including Oct-1, were not affected and no significant cell death was observed by a dye-exclusion assay and phase-contrast microscopy at inhibitor concentrations preventing NF-αB activation. Leupeptin, antipain and the trypsin inhibitor tosyl-Lys-methylester were not effective, even at high concentrations.

TABLE 1

THE EFFECT OF VARIOUS PROTEASE INHIBITORS ON THE ACTIVATION OF NF-κB

| Protease inhibitor | Jurkat | 70Z/3 |
|---|---|---|
| Tosyl-Phe-chloromethylketone (TPCK) | 20* | 20 |
| Benzyloxycarbonyl-Leu-Tyr-chloromethylketone (2LYCK) | 10 | 10 |
| Tosyl-Lys-chloromethylketone (TLCK) | 100 | 100 |
| 3,4-Dichloroisocoumarin | 20 | ND |
| N-Benzoyl-L-Tyr-ethylester (BTEE) | 1,000 | ND |
| N-Acetyl-DL-Phe-β-Naphthylester (APNE) | 200 | 200 |

Jurkat and 70/3 cell cultures were incubated with the listed compounds at various concentrations for 30 minutes and then stimulated by either TNF-α (Jurkat cells) or PMA (7OZ/3). Total cell or nuclear extracts were prepared and analyzed for NF-κB activity using EMSA.

*$IC_{90}$ (μM), 90% inhibitory concentration; ND, not determined. Jurkat T cells were cultured as described (Schreck, R., *et al., EMBO J.*, 10:2247, 1992). All compounds (Sigma) were dissolved in DMSO and cells pretreated at various concentrations for 30 min. EMSA was done as described (Henkel, *et al., Cell,* 68:1121, 1992).

The effect of tosyl-Phe-chloromethylketone (TPCK) was investigated in more detail. Cell culture, extract preparation, EMSA, SDS-PAGE and Western blotting were as described above. 70Z/3 cells were pretreated with 25 μM TPCK or TLCK (Sigma) dissolved in dimethylsulphoxide (DMSO). Control cultures received an equivalent amount of DMSO. Cell cultures were treated with the ammonium salt of PDTC (Sigma).

FIGURE 3a shows the effect of TPCK on activation of NF-κB and stability of IκBα. 70Z/3 cells were stimulated with PMA for the indicated times in the absence (left panel) or presence of 25 μM TPCK (1 hour pretreatment). Cell extracts were analyzed for NF-κB activity by EMSA (upper panels) and for IκBα levels by Western blotting (lower panel). An arrow indicates the position of the 38K IκBα band in Western blots. The faint lower band is nonspecific because its abundance was strongly reduced when affinity-purified antibody as used. The position of the NF-κB-DNA complex in the sections of fluorograms is indicated by filled arrowheads. (b) The effect of TPCK on the activation of NF-κB by IL-1β, LPS and PMA in 70Z/3 cells; Co, Control cells. Cells were treated with TPCK 10 minutes before stimulation (lanes 6-8) or, for 10 min, following treatments of cells for 30 minutes with IL-1 (L) and PMA (P), and for 60 minutes with LPS (L) (lanes 10-12). Total cell extracts from control (lanes 1-4) and TPCK-treated cells (lanes 5-12) were analyzed for NF-κB activity by EMSA. (c) The effect of TLCK on the activation of NF-κB by PMA. 70Z/3 cells were left untreated (lane 1) or treated for 10

minutes with either 25 μM TPCK (lane 2) or 25 μM TLCK (lane 3) followed by addition of PMA. Cell extracts were analyzed by EMSA for NF-κB activity. (d) The effect of the antioxidant PDTC. Cells were treated for the indicated periods of time with PMA in the absence or presence of 100 μM PDTC added 1 hour before stimulation. Cell extracts were analyzed for NF-κB activity by EMSA (upper panels) and for IκBα levels by Western blotting (lower panel).

Treatment of cells with 25 μM TPCK fully inhibited the induction of NF-κB activity (FIGURE 3a, upper panels) as well as the decay of IκB-α in response to PMA (lower panels). The half-maximal inhibitory concentration ($IC_{90}$) of TPCK was about 8 μM. The protease inhibitor prevented the activation of NF-κB by IL-1β and LPS in 70Z/3 cells (FIGURE 3b, lanes 6 and 7) and by TNF-α in various other cells lines. TPCK did not strongly interfere with NF-κB activity when added after stimulation with PMA, IL-1 or LPS but seemed to arrest a further activation of the factor (FIGURE 3b, lanes 10-12). It is unlikely that TPCK acted on protein kinase C (PKC) because IL-1 and TNF-α activate NF-κB independently of PMA-inducible PKC isoenzymes (Bomsztyk, *et al., Cell. Regul.* 2:329, 1991; Schreck, R., *et al., J. Biol. Chem.*, 265:8339, 1990). Furthermore, TPCK did not interfere with early signalling events of the TNF receptor. TLCK, an inhibitor of trypsin-like serine proteases, which is highly related in structure and chemical activity to TPCK, could not prevent activation of NF-κB at a concentration of 25 μM (FIGURE 3c, lane 3), but only at 100 μM (Table 1). The pharmacological data strongly support the requirement of proteolytic degradation of IκBα for the activation of NF-κB. The inhibitor profile suggests the involvement of a serine protease with chymotrypsin-like specificity, which we will refer to as IκBα protease. Although it cannot be ruled out that the protease inhibitors interfered with another proteolytic step located upstream from the IκBα protease, this appears unlikely in view of the diversity of NF-κB activators, which might have very few upstream pathways in common.

Reactive oxygen intermediates (ROIs) seem to play a role as messengers in the activation of NF-κB by many inducing conditions. A potent antioxidant inhibitor of NF-κB activation is pyrrolidimedithiocarbanate (PDTC). No significant decay of IκBα or activation of NF-κB was seen after PMA stimulation in 70Z/3 cells pre-treated with 100 μM PDTC, (FIGURE 3d), suggesting the proteolysis of IκBα is controlled by ROIs. PDTC does not interfere with the PMA-induced membrane association and kinase activity of PKC, which argues against a direct phosphorylation of IκBα by PKC in intact cells.

## EXAMPLE 3

## NF-κB ACTIVATION IN JURKAT CELLS REQUIRES COSTIMULATION AND INVOLVES THE PHOSPHORYLATION AND DEGRADATION OF IκBα

Physiological activation of T lymphocytes requires costimulation through the TCR/CD3 receptor and CD28 (Fraser, J.D., *et al., Immunol. Today*, 14:357-362, 1993; Linsey, P.S., *et al., Annu. Rev. Immunol.*, 11:191-212, 1993; Umlauf, S.W., *et al., Immunol. Rev.*, 133:177-197, 1993), which can be mimicked by suboptimal PMA concentrations in combination with the engagement of one receptor or $Ca^{++}$ ionophore (Fraser, J.D., *et al., supra*; Linsley, P.S., *et al., supra*; Umlauf, S.W., *et al., supra*; Crabtree, G.R., Science, 243:355-361, 1989; Su, B., *et al., Cell*, 77:727-736, 1994). Previous studies indicated that NF-κB activation is enhanced by costimulation (Sen, R., *et al., Cell*, 47:921-928, 1986; Lenardo, M.J., *et al., Cell*, 58:227-229, 1989; Mattila, P.S., *et al., EMBO J.*, 9:4425-4433, 1990; Frantz, B., *et al., EMBO J.*, 13:861-870, 1994).

The steps involved in the activation of NF-κB, include IκBα elimination through the appearance of the nuclear DNA binding activity, and the transcriptional activity. NF-κB activation was measured through the reporter activity of the HIV-LTR promoter, which is largely controlled by NF-κB in conjunction with the HIV Tat protein (Nabel, G., *et al., Nature*, 326:711-713, 1987). Jurkat cells were stimulated with the following reagents: (P), PMA (phorbol-12 myrislated 13-acetate), 10 ng/ml; (I), A23187 $Ca^{++}$ ionophore, 1μM; (3), anti-CD3 (OKT3), 5μg/ml; (28) anti-CD28 (monoclonal antibody 9.3), 3 μg/ml; (P/I), PMA + $Ca^{++}$ ionophore in IκBα transfected Jurkat cells; (NS) non-stimulated (0.1%, DMSO only). Jurkat cells were transfected by electroporation (Su, B., *et al., supra*) with 1 μg HIV-LTR-LUC (Israel, S., *et al., Gene*, 104:139-145, 1991) and 4 μg PBC-TAT. Control cultures were co-transfected with 8 μg Rc-CMV-IκBα (Zabel, U., *et al., EMBO J.*, 12:201-211, 1993). Following 30 hours transfection, the cells were stimulated for 8 hours and luciferase activity was determined using the Promega luciferase assay system.

Subcellular protein extracts were prepared essentially according to Beg, A.A. *et al., supra*. Following 20 minutes stimulation of Jurkat cells, cytosolic extracts (50 μg/point) were analyzed by Western blot, and nuclear extracts (5 μg/point) by EMSA, as described (Henkel, T., *et al., Nature*, 365:182-185, 1993).

Rabbit anti-IκBα was prepared against the GST-IκBα fusion protein by standard methods. The specifity of the antibodies was confirmed in competition assays using recombinant IκBα protein.

FIGURE 4 shows that neither $Ca^{++}$ ionophore alone, nor ligation of either CD3 or CD28, induced reporter

activity, while PMA alone at 10 ng/ml enhanced the basal activity by only two-fold. On the other hand, a combination of PMA with soluble anti-CD3 antibodies, soluble anti-CD28 antibodies or $Ca^{++}$ ionophore, enhanced reporter activity by 4 to 9 fold (FIGURE 4a). The costimulated activity of the HIV-promoter was clearly NF-κB dependent as attested to by the finding that it was completely blocked by overexpression of the IκBα co-transfected with the reporter plasmid (FIGURE 4a, $/P/I).

The costimulation requirements of NF-κB were also evident in an electromobility shift assay (EMSA) (FIGURE 4b). The upper section of the EMSA shows the NF-κB complex composed of RelA (p65) and NF-κB (p50) (arrow). Whereas single stimuli were barely sufficient to induce NF-κB activity in EMSA, combinations of PMA with either $Ca^{++}$ ionophore, anti-CD3 or anti-CD28 and costimulation with the two antibodies enhanced the NF-κB DNA binding activity 10 to 20 fold over the level induced by a single stimulus (FIGURE 4b).

Parallel to NF-κB activity, the fate of IκBα following cell treatment with the various stimuli was investigated. Single stimuli which failed to activate NF-κB did not affect the migration or intensity of the IκBα signal in a Western blot. Jurkat cells were treated for 8 minutes with the indicated stimuli as in FIGURE 4a.

In contrast, T cell costimulation resulted in the reduced intensity of the IκBα signal and the appearance of a slow migrating form of IκBα (FIGURE 4c). Alkaline phosphatase treatment of cytosolic extracts from stimulated cells reversed the slow migration of the novel IκBα band, indicating it was a phosphorylated form of IκBα. The arrow indicates the IκBα non-modified band. A non-specific band of lower molecular weight appears in the bottom of the blot and is not affected by cell stimulation.

## EXAMPLE 4

### INHIBITION OF IκBα PHOSPHORYLATION BLOCKS ITS INDUCIBLE DEGRADATION

On the basis of the results in Example 3, phosphorylation of IκBα, in parallel to NF-κB activation, is induced according to the physiological requirements for T cell activation. Cell stimulation and extract preparation was as described above. Jurkat cells were treated prior to stimulation with 25 μM TPCK (Boehringer) or 400 ng/ml CsA (Sandoz) for 30 min, or with 100 μM PDTC (Sigma) for 1 hour. Duration of Jurkat cell stimulation with PMA (10 ng/ml) and $Ca^{++}$ ionophore (1μM) was determined by Western blot analysis. IκBα arrow indicates the position of the non-modified IκBα and the * arrow, the position of the modified IκBα form. Duration of Jurkat stimulation with PMA and $Ca^{++}$ ionophore was as indicated.

Kinetic studies of the IκBα modification process revealed that concomitant with the appearance of phosphorylated IκBα, the non-modified IκBα band diminished (FIGURE 5a). Upon densitometry analysis, the two IκBα signals at 7 min. post stimulation equaled the single IκBα signal from non-stimulated cells. Likewise, between 7 and 9 min., the intensity of the phosphorylated IκBα signal equaled the consecutive signal loss of the non-modified band (FIGURE 5a). These features show that the decay of the non-phosphorylated IκBα signal occurs subsequent to its modification, and that the decay of the phosphorylated band is due to degradation.

To test the hypothesis that IκBα degradation requires its prior phosphorylation, three different reagents were used which have been shown to block NF-κB activity: Tosyl-Phe-cloromethylketone (TPCK) (Henkel, T., *et al.*, *supra*), pyrrolidinedithiocarbamate (PDTC) (Henkel, T., *et al.*, *supra*; Scherck, R., *et al.*, *J. Exp. Med.*, 175:1181-1194, 1992; Sun, SC., *et al.*, *Science*, 259:1912-1915, 1993) and cyclosporine A (CsA) (Su, B., *et al.*, *supra*).

TPCK, a serine protease inhibitor and PDTC a powerful antioxidant blocked both the inducible modification and degradation of IκBα completely (FIGURE 5b). Cyclosporine A, a powerful immunosuppressive drug and inhibitor of the phosphatase calcineurin (Schriber, S.L., *Cell*, 70:365-368, 1992), abolished most of the inducible IκBα phosphorylation and degradation (FIGURE 5b). The phosphorylation of IκBα in cells treated with CsA prior to stimulation was initiated at a lower efficiency and barely developed during the 20 min. assay. The fact that all three reagents, each with its individual mode of action, blocked both the inducible IκBα modification and its degradation, indicates that phosphorylation is a prerequisite for IκBα degradation.

## EXAMPLE 5

### IκBα PHOSPHORYLATION IS NOT SUFFICIENT FOR NF-κ ACTIVATION

Since the stability of IκBα was shown to be enhanced by complexing to Rel A (p65), it was suggested that modification of IκBα could lead to its dissociation and destabilization (Sun, S.C., *et al.*, *supra*, 1993; Scott, M.L., *et al., Genes& Dev.*, 7:1266-1276, 1993; Rice, N.R., *et al., EMBO J.*, 12:4685-4695, 1993; Chiao, P.J., *et al., Proc. Natl. Acad. Sci.* USA, 91:28-32, 1994). Once dissociation is induced through IκBα phosphorylation, the released Rel proteins would be free to translocate to the nucleus and bind to the kB site. Subsequent degradation of the released IκBα would complement the dissociation step ensuring its irreversibility. The previous Examples (1 and 2) led to the preliminary characterization of the IκBα protease as a serine protease likely related to chymotrypsin (Henkel, T., *et al., supra*). A candidate cytoplasmic protease which could eliminate IκBα following stimulation is the ubiquitous 700 kD multisubunit proteosome with its distinctive chmotrypsin-like activity (Vinitsky, A., *et al., Biochemistry*, 31:9421-9428 (1992)).

N-acetyl-Leu-Leu-Norleucinal (AcLLnL) is a reversible non-alkylating proteosome inhibitor not likely to disturb post-translation modification events. Therefore, the effect of AcLLnL on IκBα processing was studied. Immunoprecipitation (IP) was performed by incubating 500 μg cytosolic extract in 200 μl Hepes buffer (20 mM), pH 7.6, containing 10 mM KCl, protease and phosphatase inhibitors (Su, *et al.*, *supra*), with 3 μl antiserum. Following 18 hours incubation at 0°C, IPs were recovered from protein A Sepharose beads and subjected to Western blot analysis with anti-IκBα. Anti-Rel A serum was a rabbit polyclonal antiserum prepared against the C-terminus portion of Rel A. In FIGURE 6a, cells were pretreated for 4 hours with 50 μg/ml AcLLnL, stimulated with PMA (10 ng/ml) and $Ca^{++}$ ionophore (1 μM) for the time intervals indicated and analyzed by Western blot. The arrow indicates the non-modified IκBα. AcLLnL and other peptide aldehydes utilized in the Examples were synthesized by standard methods known in the art.

Pretreatment of Jurkat cells with the proteosome inhibitor, AcLLnL, did not interfere with IκBα phosphorylation, but completely blocked IκBα degradation (FIGURE 6a). Some variations were observed in the ratio of the two IκBα forms, with accumulation of the phosphorylated form peaking 20 minutes after cell stimulation. Nevertheless, the sum of the two forms remained constant, as determined by densitometry analysis.

Nuclear extracts corresponding to the two late time points were tested for the effect of AcLLnL on NF-κB activity (FIGURE 6b). In FIGURE 3b, the duration of stimulation in the presence (+) or absence (-) of AcLLnL is indicated. In the upper section of the EMSA autoradiogram, the arrow indicates the NF-κB complex. In the presence of the protease inhibitor, nearly all of the inducible NF-κB activity was abolished irrespective of the phosphorylation status of IκBα. Hence, IκBα phosphorylation per se is not sufficient to enable the nuclear translocation of NF-κB, but must be accompanied by its proteolytic elimination.

Although not wanting to be bound by a particular theory, these results could be explained by two different models. The first entails phosphorylation dependent IκBα-Rel dissociation and subsequent destabilization of IκBα, with proteolysis preventing IκBα reassociation. If so, in activated cells, cytoplasmic complexes would not be expected to contain phosphorylated IκBα and Rel A. Alternatively, phosphorylation could tag IκBα for proteolysis. In the event, it should be possible to detect a transient complex containing Rel A and phosphorylated, but intact IκBα.

The composition of the Rel A complex was assessed by immunoprecipitation with anti-Rel A serum (FIGURE 6c, lane 4) or control serum (C, lane 3) followed by Western blot analysis with anti IκBα. FIGURE 6c shows that IκBα phosphorylation does not lead to its dissociation from the Rel complex. Lanes 2 and 4 show immunoprecipitates (IP) with anti-Rel A serum; lane 3, IP with control anti-cAbl serum; lanes 1 and 5 are reference points (prior to IP) corresponding to the 7 minute point from FIGURE 4a and the 30 minute point from FIGURE 6a, respectively. In the presence of phosphatase inhibitors, the 18 hours immunoprecipitation of cytoplasmic extracts from an early time point post stimulation with anti-Rel A, resulted in the recovery of equal amounts of phosphorylated and non-phosphorylated IκBα in a complex with Rel A. Similar results were obtained with another anti-Rel A serum (Vinitsky, A. *et al.*, *supra*). In the presence of AcLLnL, Rel A immunocomplexes from late (FIGURE 6c, lane 2) or early post-stimulation time-points, contained intact phosphorylated IκBα. It is therefore clear that the affinity of IκBα to Rel A does not diminish following its induced phosphorylation. The complex between Rel A and the modified IκBα persists as long as it is not subject to the cell's proteolytic machinery (FIGURE 6, a+c).

## EXAMPLE 6

## INHIBITION OF IκBα DEGRADATION BY PEPTIDE ALDEHYDES

The results in Example 5 showed that the peptide aldehyde, AcLLnL, effectively inhibited IκBα degradation, therefore other peptide aldehydes were examined for inhibition of IκBα degradation.

Human HeLa S3 or Hep G2 cells were pretreated for 4 hours with the indicated peptide aldehyde prior to simulation with either TNFα (20ng/ml), IL-1(20 ng/ml) or PMA (100 μg/ml). The cells were harvested 20 minutes after induction and the extent of IκB degradation was examined via Western blot analysis using IκB specific antisera. FIGURE 7 summarizes the ability of related peptide aldehydes to block IκB degradation induced by a variety of agents which activate NF-κB. (+++) indicates greater than 85% inhibition, (++) indicates greater than 60% inhibition and (-) indicates no inhibition of IκB degradation was observed.

Benzyloxycarbonyl-Leu-Leu-phenylalaninal (ZLLF) was the most potent inhibitor of proteolysis of IκBα, followed by AcLLnL and AcALLnL. Substitution of the third amino acid with methionine or arginine failed to inhibit IκBα degradation.

## EXAMPLE 7

## PEPTIDE ALDEHYDES INHIBIT TRANSCRIPTION FROM NF-κB DEPENDENT PROMOTORS *IN VIVO*

The peptide aldehyde, AcLLnL was examined for inhibition of transcription from NF-κB-dependent promoters *in vivo*. Human Hep G2 cells were electroporated (220V, 960uF) with either 2X kB-LUC (J16-LUC) or RSV-LUC (10μg/0.5 x $10^7$ cells). 22 hours after electroporated, the cells were treated with the indicated peptide aldehyde (200 μM) for 3 hours, and subsequently stimulated with TNFα (20ng/ml) where indicated. The cells were then harvested 6 hours post-induction and the level of luciferase activity was determined. FIGURE 8 shows the results.

This experiment demonstrates that AcLLnL selectively inhibits transcription of κB-dependent promoters *in vivo*. Furthermore, the related peptide aldehyde, AcLLR, had no effect on κB-dependent transcriptional activation.

## EXAMPLE 8

## DOSE RESPONSE OF NF-κB DNA BINDING AND IκBα DEGRADATION TO THE PEPTIDE ALDEHYDE PROTEOSOME INHIBITORS Z-LLFCHO AND Ac-LLnLCHO

Two peptide aldehydes, one with phenylaline substitution (ZLLF) and one with norleucinal (AcLLnL) were examined for NFκB DNA binding in a dose response assay.

Hela cells were pretreated for 5 hours with the indicated concentration of peptide aldehyde inhibitor or left untreated and then stimulated with TNFα (15ng/ml) for 20 minutes. The cells were then harvested for whole cell extract preparation. Extracts were analyzed for NF-κB DNA binding activity by electrophoretic mobility shift assay (EMSA) and relative IκBα protein levels were measured by Western blot analysis as shown in FIGURE 9.

The results show that at concentrations of 1μM to 200 μM, AcLLnL and ZLLF inhibited TNFα induced NF-κB activity in a dose-dependent manner. Similarly, IκBα degradation was minimal in the presence of the peptide aldehydes.

The inset at the bottom of FIGURE 9 shows the $IC_{50}$ for ZLLF and AcLLnL as determined by densitometric scanning of the EMSA and Western blots. $IC_{50}$ for ZLLF was 3.4 μM and 12.1 μM for AcLLnL.

Therefore, the ultimate role of inducible IκBα phosphorylation is, most likely, to tag IκBα for degradation. A cytoplasmic protease, such as the proteosome, would recognize the tagged IκBα within the Rel complex and degrade it.

The foregoing is meant to illustrate, but not to limit, the scope of the invention. Indeed,those of ordinary skill in the art can readily envision and produce further embodiments, based on the teachings herein, without undue experimentation.

## Claims

**1.** A method of identifying a composition which affects degradation of IκBα comprising:
a) incubating components comprising the composition, phosphorylated IκBα, and a chymotrypsin-like serine protease under conditions sufficient to allow the components to interact; and
b) measuring the effect on the protease caused by the composition.

**2.** The method of claim 1, wherein NF-κB is further comprised in the incubated components.

**3.** The method of claim 2, wherein the IκBα is bound to the NF-κB.

**4.** The method of any one of claims 1 to 3, wherein the chymotrypsin-like serine protease is chymotrypsin.

**5.** The method of any one of claims 1 to 4, wherein the effect is inhibition of the protease.

**6.** The method of any one of claims 1 to 4, wherein the effect is stimulation of the protease.

**7.** A method of identifying a composition which affects degradation of IκBα comprising:
a) incubating components comprising the composition, an inducer of NF-κB, and an indicator cell; and
b) detecting NF-κB activity.

**8.** The method of claim 7, wherein the indicator cell is recombinantly modified to contain at least one copy of the κB binding motif.

**9.** The method of claim 8, wherein the κB binding motif is operably linked to a reporter gene.

**10.** The method of claim 9, wherein the reporter gene is selected from the group consisting of β-lactamase, chloramphenicol acetyltransferase (CAT), adenosine deaminase (ADA), aminoglycoside phosphotransferase (neo, G418), dihydrofolate reductase (DHFR), hygromycin-B-phosphotransferase (HPH), thymidine kinase (TK), β-galactosidase (β-gal), and xanthine guanine phosphoribosyltransferse (XGPRT).

**11.** A pharmaceutical composition comprising a therapeutically effective amount of an inhibitor of a chymotrypsin-like serine protease which degrades IκBα.

**12.** The pharmaceutical composition of claim 11, wherein the inhibitor is a peptide aldehyde.

**13.** The pharmaceutical composition of claim 12, wherein the peptide aldehyde has the formula,

$$R_1R_2LLR_3\text{-CHO}$$

wherein $R_1$ is an optional amine protective group; L is leucine; $R_2$ is optional and has the formula

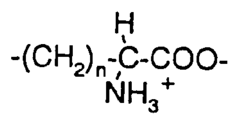

wherein
n is 1-5 and wherein $(CH_2)_n$ is a branched or straight chain alkyl; and $R_3$ is an optional hydrophobic amino acid, except for methionine.

**14.** The pharmaceutical composition of claim 13, wherein $R_3$ is selected from the group consisting of alanine, valine, leucine, isoleucine, proline and phenylalanine.

**15.** The pharmaceutical composition of claim 13 or 14, wherein the amine protective group is selected from the group consisting of N-acetyl, benzyloxycarbonyl and butyl.

**16.** The pharmaceutical composition of claim 13, wherein the peptide aldehyde is selected from the group consisting of N-acetyl-Leu-Leu-Norleucinal (AcLLnL), N-acetyl-Ala-Leu-Leu-Norleucinal-CHO (AcALLnL) and benzyloxycarbonyl-Leu-Leu-phenylalaninal (ZLLF).

**17.** Use of an inhibitor of a chymotrypsin-like serine protease which degrades IκBα for the preparation of a pharmaceutical composition of any one of claims 11 to 16 for treating an immunopathological disorder associated with NF-κE gene activation in a subject.

**18.** Use of claim 17, wherein the immunopathological disorder is selected from the group consisting of acquired immunodeficiency disorder (AIDS), toxic shock syndrome, allograft rejection, ultraviolet and radiation responses, and cachexia associated with advanced cancer.

**19.** Use of an inhibitor of a chymotrypsin-like serine protease which degrades IκBα for the preparation of a pharmaceutical composition of any one of claims 11 to 16 for modulating the activation of a virus associated with NF-κB transactivation.

**20.** Use of claim 19, wherein the virus is human immunodeficiency virus (HIV).

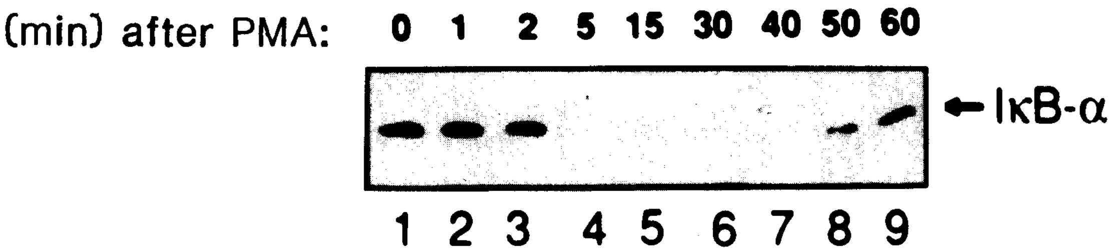
(min) after PMA: 0 1 2 5 15 30 40 50 60
IκB-α
1 2 3 4 5 6 7 8 9

FIG. 1a

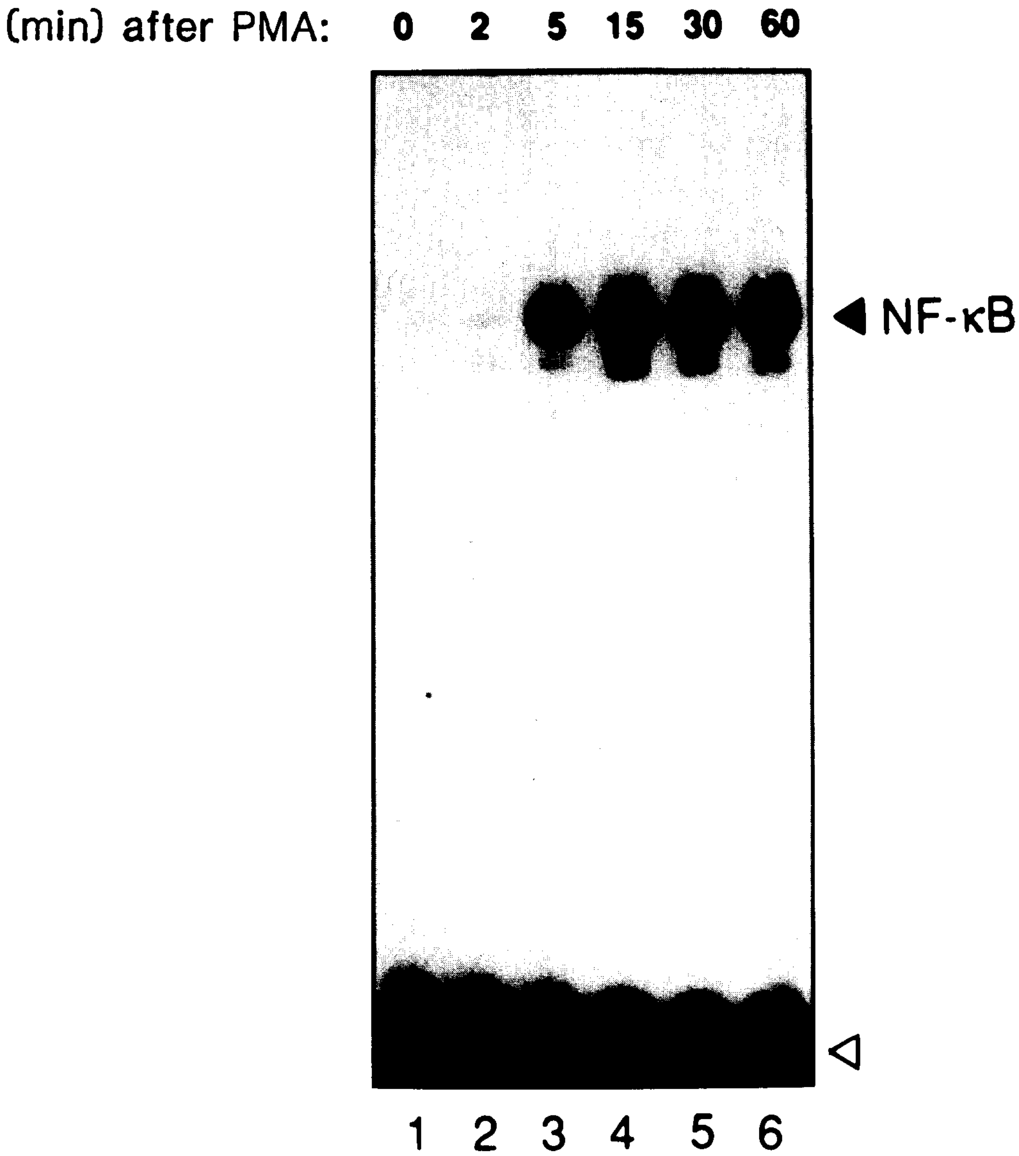
(min) after PMA: 0 2 5 15 30 60
NF-κB
1 2 3 4 5 6

FIG. 1b

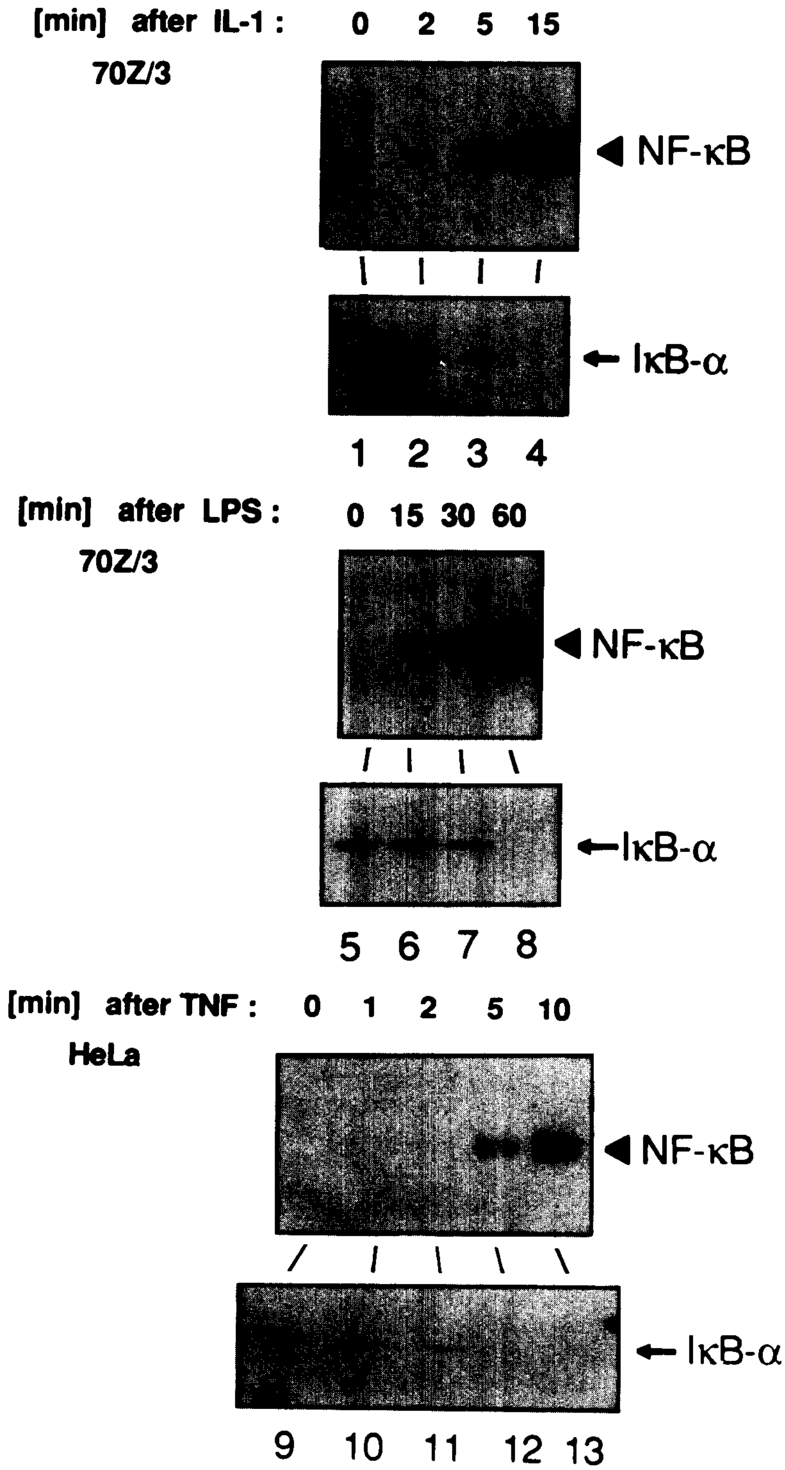
[min] after IL-1 : 0 2 5 15
70Z/3
NF-κB
IκB-α
1 2 3 4
[min] after LPS : 0 15 30 60
70Z/3
NF-κB
IκB-α
5 6 7 8
[min] after TNF : 0 1 2 5 10
HeLa
NF-κB
IκB-α
9 10 11 12 13

FIG. Ic

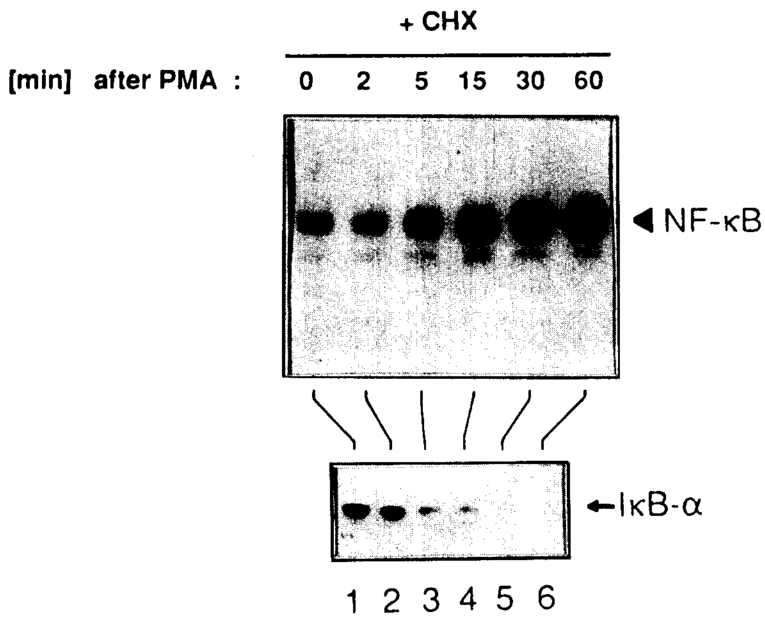
+ CHX
[min] after PMA :
0 2 5 15 30 60
NF-κB
IκB-α
1 2 3 4 5 6

FIG. 2a

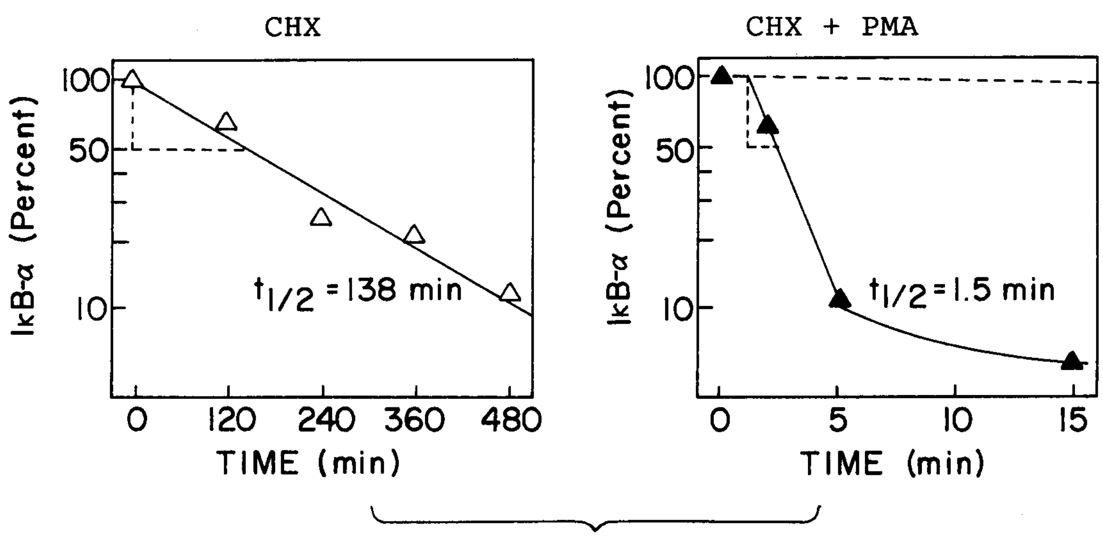
CHX
CHX + PMA
IκB-α (Percent)
100
50
10
t1/2 = 138 min
t1/2 = 1.5 min
0 120 240 360 480
0 5 10 15
TIME (min)
TIME (min)

FIG. 2b

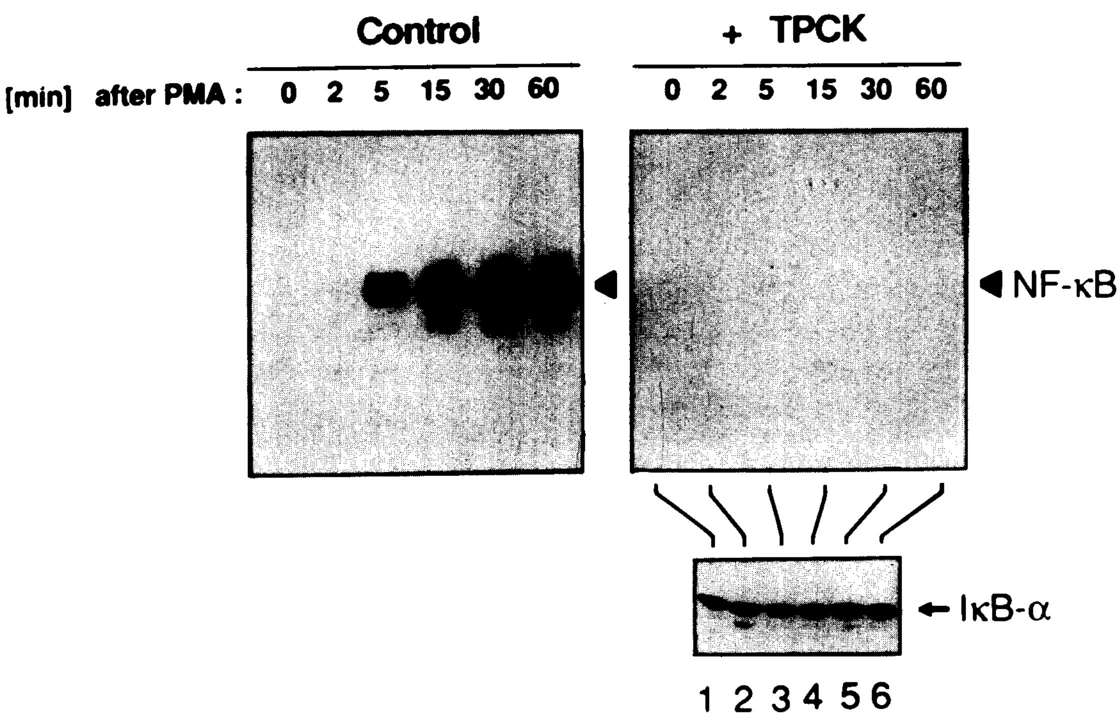
Control
+ TPCK
[min] after PMA : 0 2 5 15 30 60
0 2 5 15 30 60
NF-κB
IκB-α
1 2 3 4 5 6

FIG. 3a

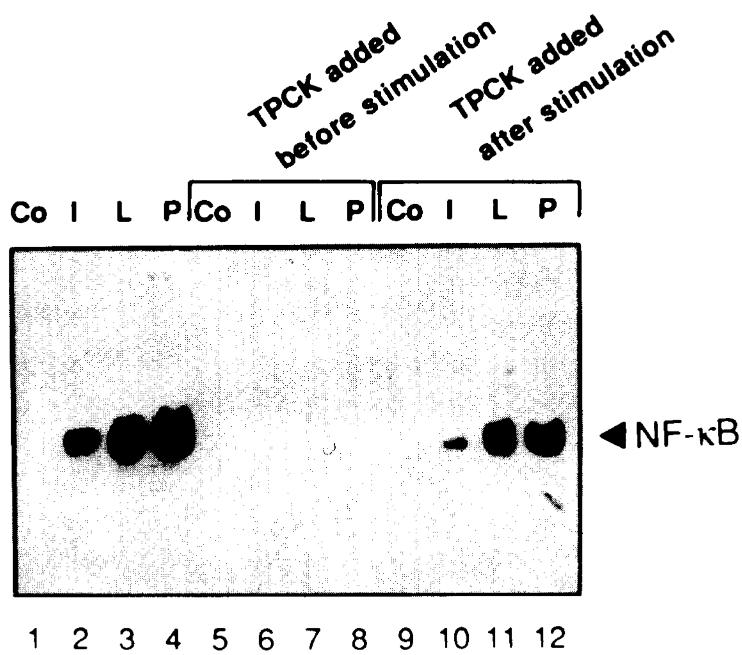
TPCK added before stimulation
TPCK added after stimulation
Co I L P Co I L P Co I L P
NF-κB
1 2 3 4 5 6 7 8 9 10 11 12

FIG. 3b

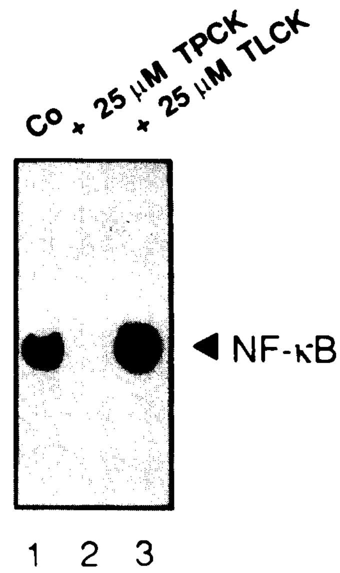
Co
+ 25 μM TPCK
+ 25 μM TLCK
NF-κB
1 2 3

FIG. 3c

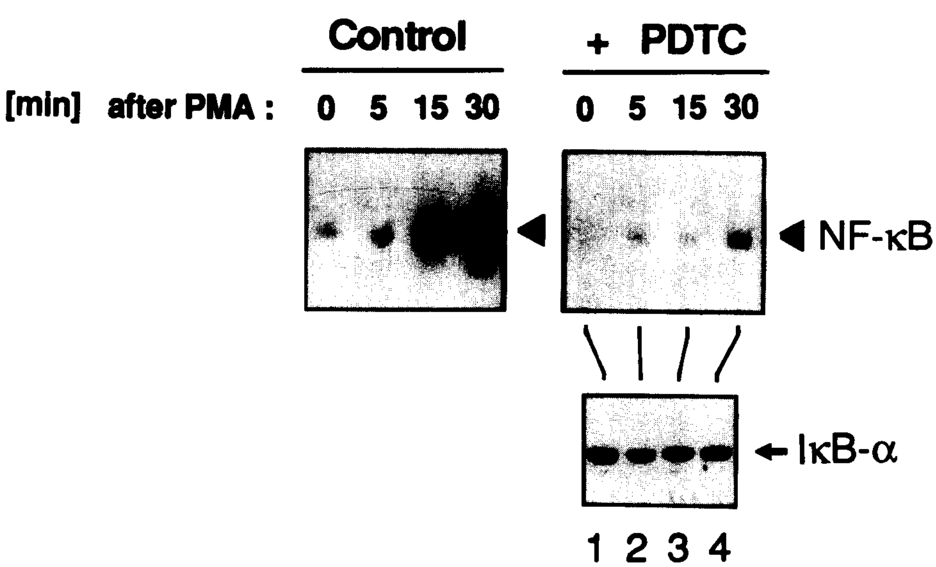
Control
+ PDTC
[min] after PMA : 0 5 15 30 0 5 15 30
NF-κB
IκB-α
1 2 3 4

FIG. 3d

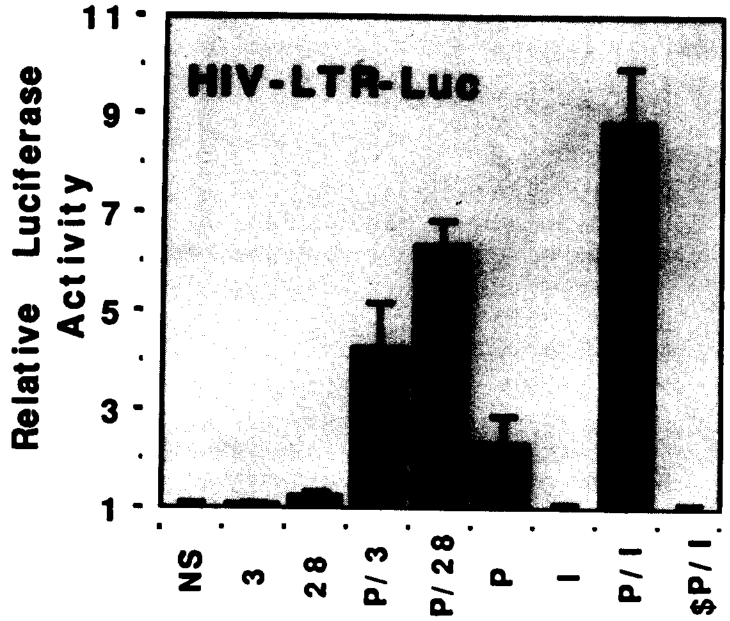


FIG. 4a

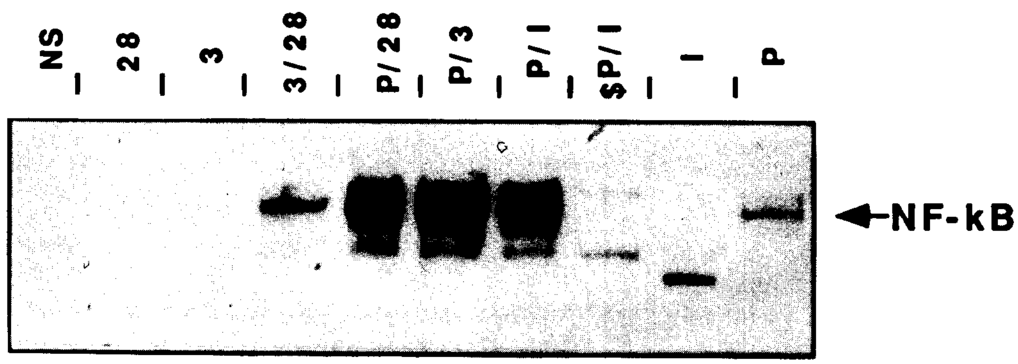


FIG. 4b

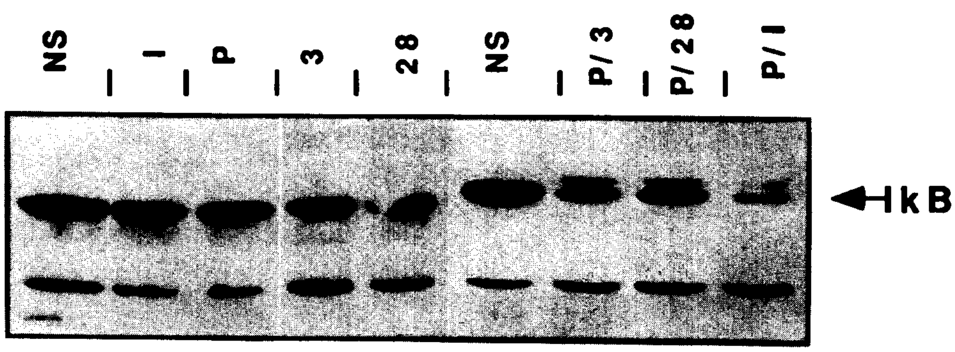


FIG. 4c

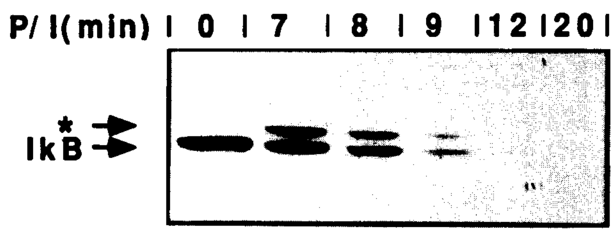
P/I(min) 0 7 8 9 12 20
IkB*

FIG. 5a

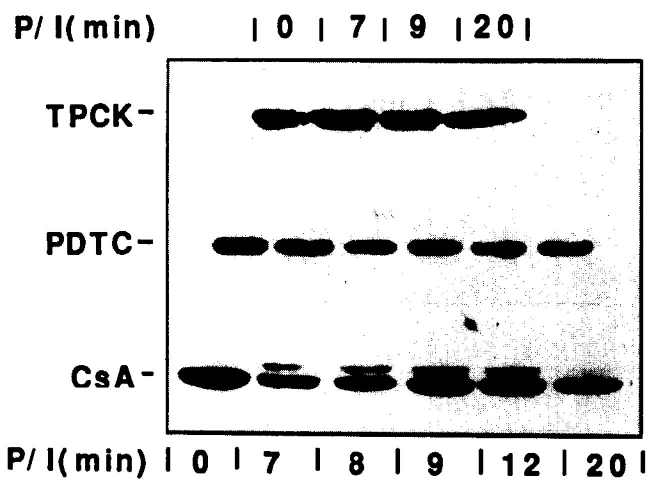
P/I(min) 0 7 9 20
TPCK-
PDTC-
CsA-
P/I(min) 0 7 8 9 12 20

FIG. 5b

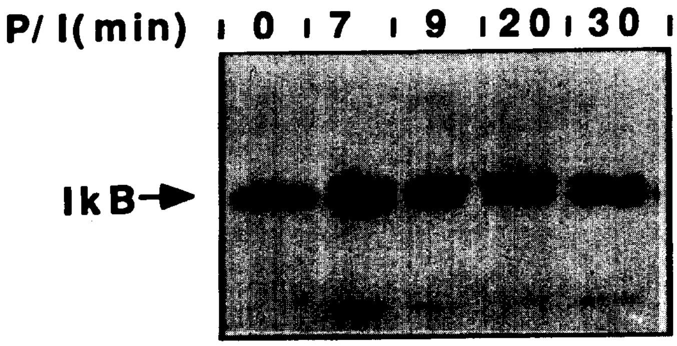


FIG. 6a

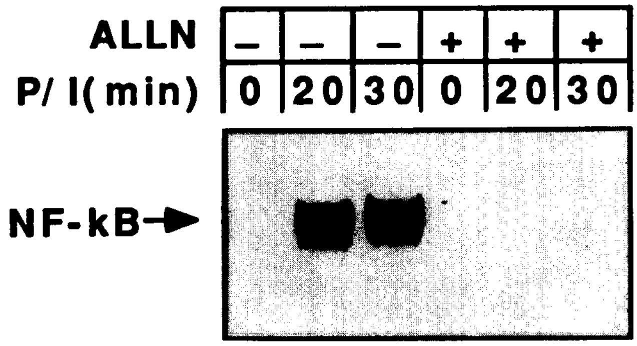


FIG. 6b

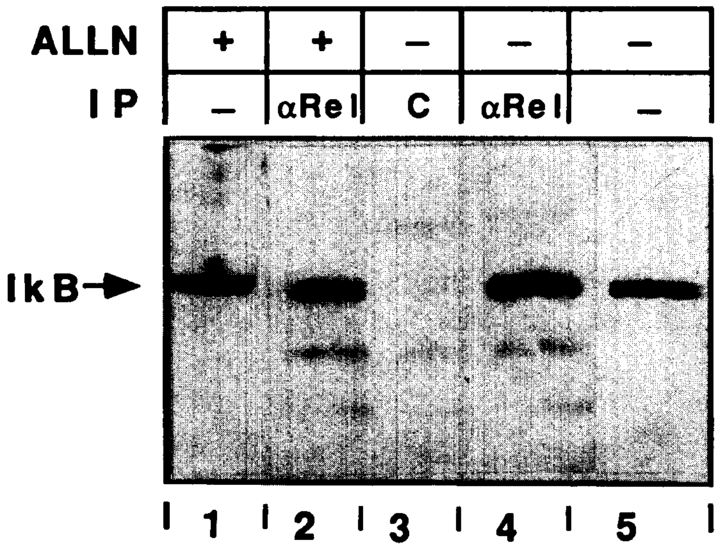


FIG. 6c

| PEPTIDE ALDEHYDE INHIBITORS: | INDUCERS OF IXBα DEGRADATION | | |
|---|---|---|---|
| | TNFα | IL-1α | PMA |
| Z-LLF-CHO | +++ | +++ | +++ |
| Ac-LLnL-CHO | ++ | ++ | ++ |
| Ac-ALLnL-CHO | ++ | ++ | ++ |
| Ac-LLM-CHO | — | — | — |
| Ac-LLR-CHO | — | — | — |
| Z-IEL-CHO | — | — | — |

Z-LLF-CHO: (benzyloxycarbonyl)-Leu-Leu-phenylalaninal

Ac-LLnL-CHO: N-acetyl-Leu-Leu-norleucinal

Ac-ALLnL-CHO: N-acetyl-Ala-Leu-Leu-norleucinal

Ac-LLM-CHO: N-acetyl-Leu-Leu-methioninal

Ac-LLR-CHO: N-acetyl-Leu-Leu-arginal

Z-IEL-CHO: (benzyloxycarbonyl)-Ile-Glu-leucinal

FIG. 7

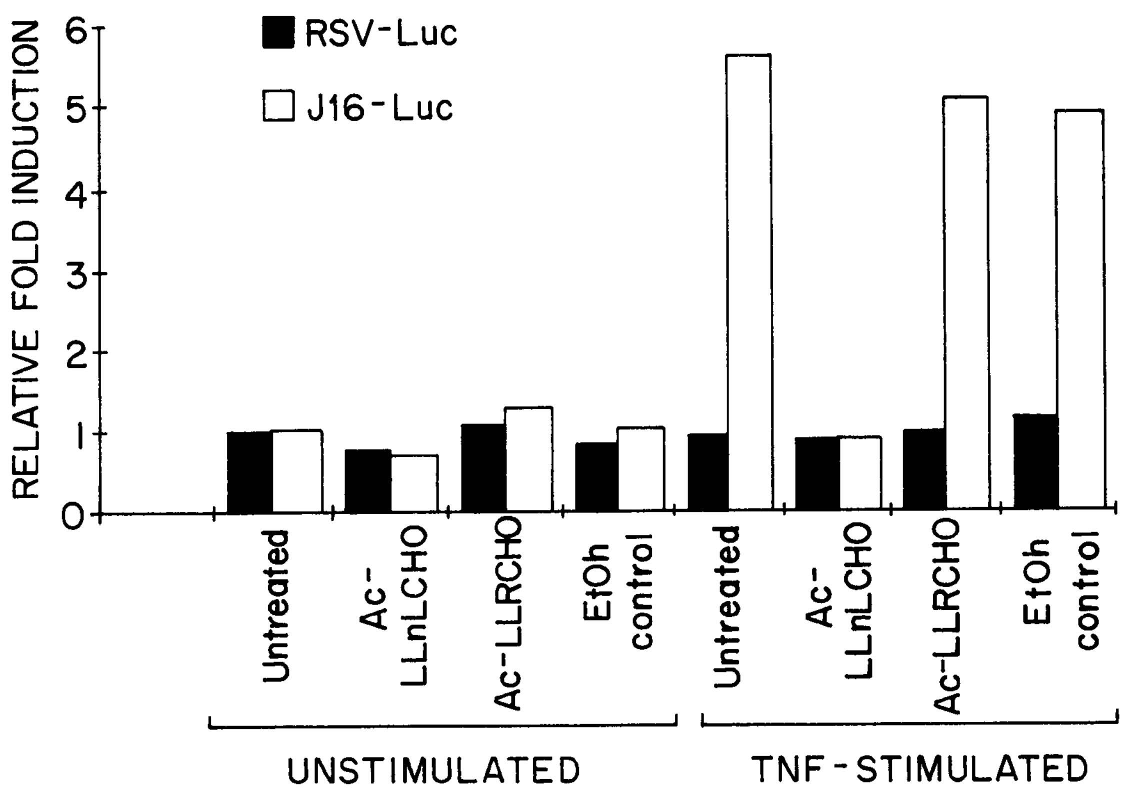
RELATIVE FOLD INDUCTION
6
5
4
3
2
1
0
RSV-Luc
J16-Luc
Untreated
Ac-LLnLCHO
Ac-LLRCHO
EtOh control
Untreated
Ac-LLnLCHO
Ac-LLRCHO
EtOh control
UNSTIMULATED
TNF-STIMULATED

FIG. 8

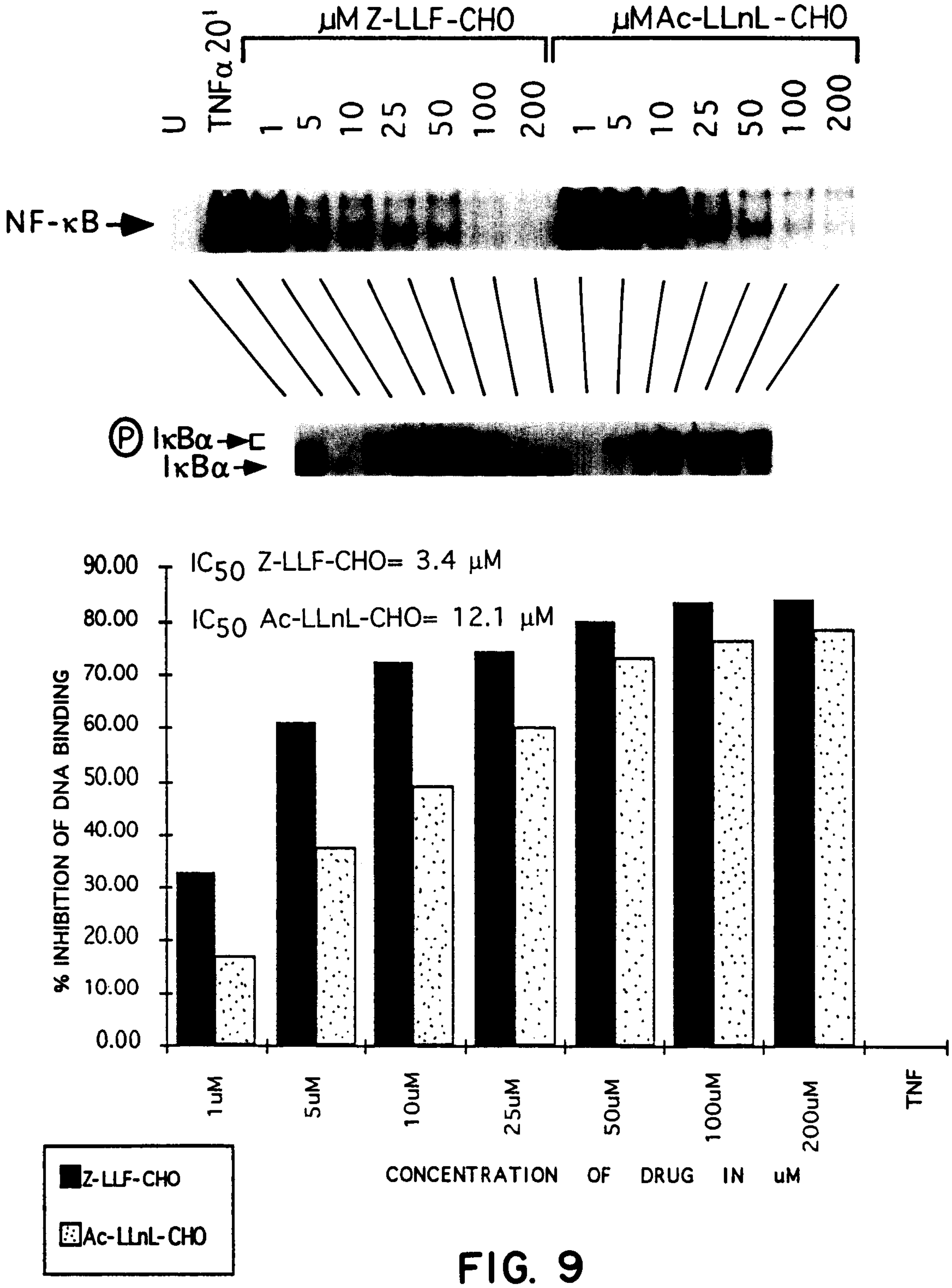
U
TNFα 20'
μM Z-LLF-CHO
1 5 10 25 50 100 200
μM Ac-LLnL-CHO
1 5 10 25 50 100 200
NF-κB
Ⓟ IκBα
IκBα
IC50 Z-LLF-CHO= 3.4 μM
IC50 Ac-LLnL-CHO= 12.1 μM
% INHIBITION OF DNA BINDING
90.00
80.00
70.00
60.00
50.00
40.00
30.00
20.00
10.00
0.00
1uM
5uM
10uM
25uM
50uM
100uM
200uM
TNF
CONCENTRATION OF DRUG IN uM
Z-LLF-CHO
Ac-LLnL-CHO

FIG. 9

European Patent Office

# EUROPEAN SEARCH REPORT

Application Number EP 94114040.2

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 6) |
| P,A | CHEMICAL ABSTRACTS, vol. 121, no. 1, 1994, July 4, Columbus, Ohio, USA S. MIYAMOTO et al. "Enhanced I-kappa B-alpha degradation is responsible for constitutive NF-kappa B acitivity in mature murine B-cell lines" page 283, no. 2 474y; & Mol. Cell. Biol 1994, 14(5), 3276-82 -- | 1-11, 17,19 | C 12 Q 1/37<br>A 61 K 38/57<br>A 61 K 38/48 |
| P,A | CHEMICAL ABSTRACTS, vol. 120, no. 25, 1994, June 20, Columbus, Ohio, USA T. MACHLEIDT et al. "Sphingomyelinase activities proteolytic I-kappa B-alpha degradation in a cell-free system" page 715, no. 321 338h; & J. Biol. Chem. 1994, 269(19), 13760-5 ---- | 1-11, 17,19 | TECHNICAL FIELDS SEARCHED (Int. Cl.6)<br>C 12 Q<br>A 61 K |

The present search report has been drawn up for all claims

| Place of search | Date of completion of the search | Examiner |
|---|---|---|
| VIENNA | 11-11-1994 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)